(19) Europäisches Patentamt
European Patent Office
Office européen des brevets

(11) EP 1 013 169 A1

(12) **EUROPEAN PATENT APPLICATION**
published in accordance with Art. 158(3) EPC

(43) Date of publication:
**28.06.2000 Bulletin 2000/26**

(21) Application number: **98940634.3**

(22) Date of filing: **31.08.1998**

(51) Int. Cl.[7]: **A01N 43/40**

(86) International application number:
**PCT/JP98/03876**

(87) International publication number:
**WO 99/11127 (11.03.1999 Gazette 1999/10)**

(84) Designated Contracting States:
**AT BE CH CY DE DK ES FI FR GB GR IE IT LI LU MC NL PT SE**
Designated Extension States:
**AL LT LV MK RO SI**

(30) Priority: **29.08.1997 JP 23365897**

(71) Applicant:
**Meiji Seika Kaisha, Ltd.**
**Tokyo 104-8002 (JP)**

(72) Inventors:
- **TERAOKA, Takeshi, Meiji Seika Kaisha, Ltd., Kouhoku-ku, Kanagawa 222-8567 (JP)**
- **KUZUHARA, Kikuko Kanagawa 248-0033 (JP)**
- **MIKOSHIBA, Haruki, Meiji Seika Kaisha, Ltd., Kouhoku-ku, Kanagawa 222-8567 (JP)**
- **MATSUMOTO, Kuniomi, Meiji Seika Kaisha, Ltd., Kouhoku-ku, Kanagawa 222-8567 (JP)**
- **IINUMA, Katsuharu, Meiji Seika Kaisha, Ltd., Odawara-shi, Kanagawa 250-0852 (JP)**
- **FUTAMURA, Takafumi, Meiji Seika Kaisha, Ltd., Odawara-shi, Kanagawa 250-0852 (JP)**
- **YASUTAKE, Tetsuya, Meiji Seika Kaisha, Ltd., Odawara-shi, Kanagawa 250-0852 (JP)**
- **SAKANAKA, Osamu, Meiji Seika Kaisha, Ltd., Odawara-shi Kanagawa 250-0852 (JP)**
- **MITOMO, Koichi, Meiji Seika Kaisha, Ltd., Odawara-shi Kanagawa 250-0852 (JP)**
- **TANIGUCHI, Makoto Osaka 596-0827 (JP)**

(74) Representative:
**Lewin, John Harvey**
**Elkington and Fife,**
**Prospect House,**
**8 Pembroke Road**
**Sevenoaks, Kent TN13 1XR (GB)**

# (54) RICE BLAST CONTROL AGENT AND WHEAT SCAB CONTROL AGENT

(57) Disclosed are rice blast and wheat leaf rust control agents and methods for controlling rice blast and wheat leaf rust. The rice blast control agent or the wheat leaf rust control agent comprises a compound represented by formula (I):

EP 1 013 169 A1

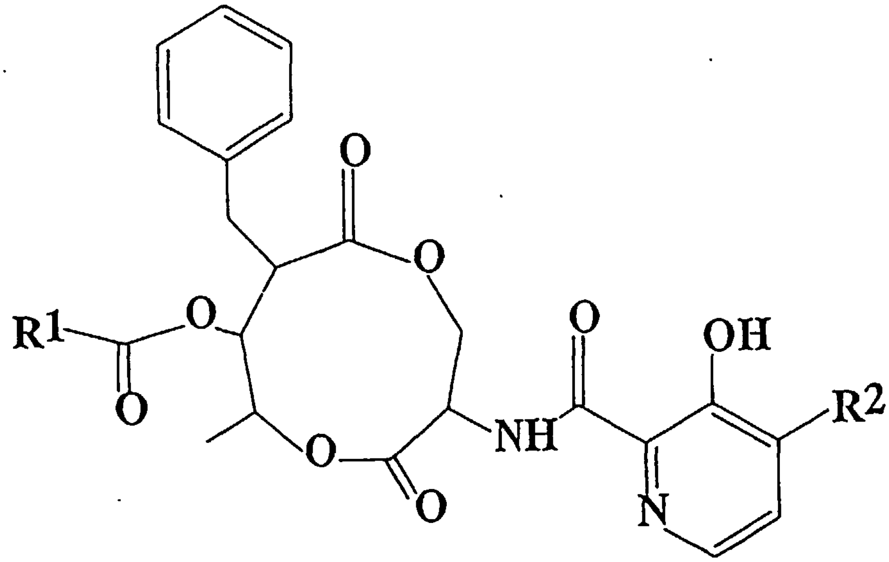

(I)

wherein $R^1$ represents alkyl group or alkenyl group and $R^2$ represents a hydrogen atom or methoxy group.
The compound represented by formula (I) has potent preventive and exterminating activity against rice blast and wheat leaf rust and does not have phytotoxicity against object plants for preventing and exterminating the diseases.

## Description

### BACKGROUND OF THE INVENTION

### Field of the Invention

The present invention relates to rice blast and wheat leaf rust control agents, and methods for preventing or exterminating these diseases.

### Background Art

Diseases of agricultural and horticultural plants greatly affect the harvest of the agricultural and horticultural plants. Therefore, the prevention or extermination of the diseases of the agricultural and horticultural plants is an important task from the viewpoints of agriculture and social economy. In particular, the prevention or extermination of blast and leaf rust, which is regarded as important in rice and wheat as main cereals, has been desired from of old.

For example, regarding chemical agents for rice blast, various chemical agents, including antibiotics, such as Kasugamycin, phospholipid biosynthesis inhibitors, such as organophosphorus compounds, and melanin pigment biosynthesis inhibitors, such as Tricyclazole and Fthalide, have been developed and put to practical use. Also for the wheat leaf rust, various chemical agents, for example, triazole EBI, such as Triadimefon, acid amide agents, such as Mepronil, and inorganic sulfur agents have been generally used.

The provision of chemical agents for the prevention or control of these diseases, however, has been still desired in the art. The development of chemical agents, which are superior in safety to environment and humans and animals, as well as in cost effectiveness, has also been desired.

On the other hand, Japanese Patent Laid-Open No. 233165/1995, J. Antibiotics 49: 639-643 (1996), and J. Antibiotics 50: 551-555 (1997) disclose a part of compounds represented by formula (I) describe below.

For example, Japanese Patent Laid-Open No. 233165/1995 discloses, as compounds of working examples, some of compounds represented by formula (I) described below, that is, a compound wherein $R^1$ represents an isopropyl group and $R^2$ represents a methoxy group (hereinafter often referred to as "UK-2A"), a compound wherein $R^1$ represents a (Z)-2-butenyl group and $R^2$ represents a methoxy group (hereinafter often referred to as "UK-2B"), a compound wherein $R^1$ represents an isobutyl group and $R^2$ represents a methoxy group (hereinafter often referred to as "UK-2C"), and a compound wherein $R^1$ represents a sec-butyl group and $R^2$ represents a methoxy group (hereinafter often referred to as "UK-2D").

Further, J. Antibiotics 50: 551-555 (1997) describes that a compound, represented by formula (I) described below wherein $R^1$ represents an isopropyl group and $R^2$ represents a hydrogen (hereinafter often referred to as "UK-3A"), has the same function and properties as described in UK-2A, UK-2B, UK-2C, and UK-2D. This literature further describes the production process of the compound.

Further, in the above publications, these compounds, by virtue of the antifungal activity, are described to be useful as an active ingredient of medical antifungal agents, agricultural and horticultural fungicides, and industrial fungicides.

### SUMMARY OF THE INVENTION

The present inventors have now found that certain conventional compounds have potent effect of preventing and exterminating rice blast and wheat leaf rust and, at the same time, do not advantageously have phytotoxic activity against plants which are objects for the extermination of diseases. The present invention has been made based on such finding.

Accordingly, it is an object of the present invention to provide a rice blast control agent and a wheat leaf rust control agent, and to provide methods for the prevention or extermination of these diseases.

The rice blast control agent or the wheat leaf rust control agent according to the present invention comprises a compound represented by formula (I):

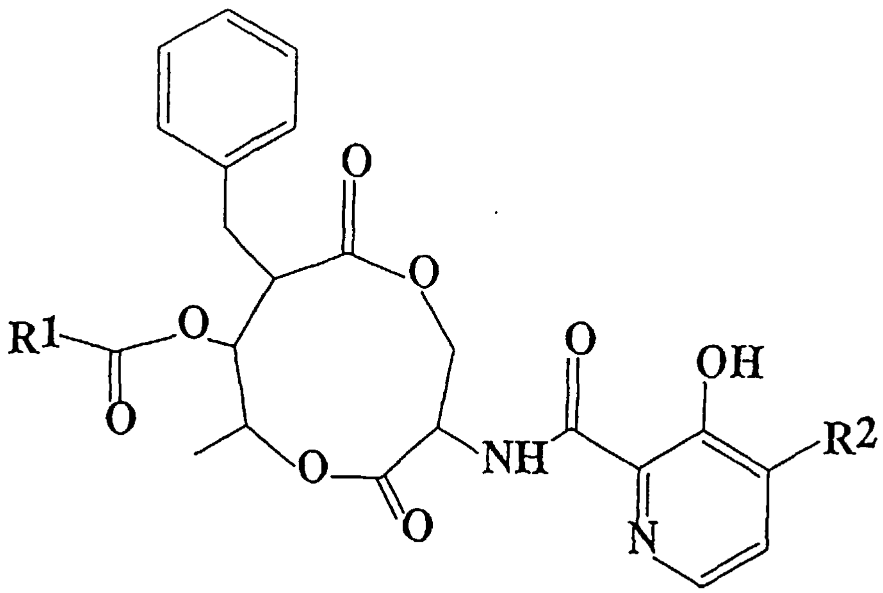

(I)

wherein

$R^1$ represents straight-chain or branched saturated aliphatic hydrocarbon group or straight-chain or branched unsaturated aliphatic hydrocarbon group; and
$R^2$ represents a hydrogen atom or methoxy group.

The method for controlling rice blast or wheat leaf rust comprises the step of applying the compound represented by formula (I) to a rice or wheat plant per se, seeds thereof, a field in which the rice or wheat plant is being grown or to be grown, or an instrument utilized in the growth of the rice or wheat plant.

DETAILED DESCRIPTION OF THE INVENTION

Deposit of microorganism

Streptoverticillium sp. SAM 2084, a microorganism capable of producing the compound represented by formula (I), is deposited under FERM BP-6446 in National Institute of Bioscience and Human-Technology, Agency of Industrial Science & Technology (1-3, Higashi 1-chome, Tukuba-shi, Ibaraki-ken, Japan). The depositor of the microorganism is Suntory Ltd. (1-40, Dojimahama 2-chome, Kita-ku, Osaka-shi, Japan). The original deposit thereof is Acceptance No. FERM P-14154 dated February 17, 1994, and the date of receipt of the request for transfer to deposit based on Budapest Treaty is August 3, 1998.

Compounds represented by formula (I)

The present invention is based on that the compounds represented by formula (I) have potent disease preventive and exterminating activity against rice blast and wheat leaf rust and, at the same time, do not have any phytotoxicity against object plants for the extermination of diseases.
As described above, the compounds represented by formula (I) are known compounds which are described to have antifungal activity and to be effective as an active ingredient of medical antifungal agents, agricultural and horticultural fungicides, and industrial fungicides. Although the compounds represented by formula (I) have antifungal activity against various fungi, surprisingly beyond the expectation of a person having ordinary skill in the art, that the compounds represented by formula (I) have especially significant preventive and exterminating activity against rice blast and wheat leaf rust.
In formula (I), the straight-chain or branched saturated aliphatic hydrocarbon group represented by $R^1$ pref-

erably has 1 to 18 carbon atoms, more preferably 1 to 6 carbon atoms, particularly preferably 3 or 4 carbon atoms. Examples thereof include alkyl groups, preferably alkyl groups having 1 to 18 carbon atoms, more preferably alkyl groups having 1 to 6 carbon atoms, particularly preferably alkyl groups having 3 or 4 carbon atoms. Specific examples thereof include methyl, ethyl, propyl, isopropyl, butyl, isobutyl, sec-butyl, pentyl, hexyl, heptyl, octyl, nonyl, undecyl, tridecyl, pentadecyl, and heptadecyl groups.

In formula (I), the straight-chain or branched unsaturated aliphatic hydrocarbon group represented by $R^1$ preferably has 2 to 18 carbon atoms with at least one double bond, more preferably 2 to 6 carbon atoms, particularly preferably 3 or 4 carbon atoms. An example thereof is alkenyl groups, preferably alkenyl groups having 1 to 18 carbon atoms, more preferably alkenyl groups having 2 to 6 carbon atoms, particularly preferably alkenyl groups having 3 or 4 carbon atoms. Specific examples thereof include vinyl, isopropenyl, (Z)-1-propenyl, (E)-1-propenyl, (Z)-2-butenyl, (E)-2-butenyl, (Z)-8-heptadecenyl, and (E)-8-heptadecenyl groups. Particularly preferred are isopropyl, (Z)-2-butenyl, isobutyl, and sec-butyl groups.

In formula (I), $R^2$ represents a hydrogen atom or methoxy group.

According to a preferred embodiment of the present invention, preferred compounds represented by formula (I) are those wherein $R^1$ represents isopropyl group, (Z)-2-butenyl, isobutyl, or sec-butyl groups and $R^2$ represents a hydrogen atom or methoxy group. Specific examples of preferred compounds include UK-2A (a compound wherein $R^1$ represents isopropyl group and $R^2$ represents methoxy group), UK-2B (a compound wherein $R^1$ represents (Z)-2-butenyl group and $R^2$ represents methoxy group), UK-2C (a compound wherein $R^1$ represents isobutyl group and $R^2$ represents methoxy group), UK-2D (a compound wherein $R^1$ represents sec-butyl group and $R^2$ represents methoxy group), and UK-3A (a compound wherein $R^1$ represents isopropyl group and $R^2$ represents a hydrogen atom).

Production of compounds represented by formula (I)

The compound represented by formula (I) can be produced from a microorganisms belonging to *Streptoverticillium*.

The microorganisms belonging to *Streptoverticillium* may be obtained by isolating Actinomycetes from a microorganism isolation source, such as soil, by a conventional method and then by selecting, from these Actinomycetes strains, strains which can produce the compounds represented by formula (I).

One example of the microorganisms, which can produce the compounds represented by formula (I), is an Actinomycetes which has been designated as *Streptoverticillium* sp. SAM 2084 and deposited under Acceptance No. FERM P-14154 dated February 17, 1994 in National Institute of Bioscience and Human-Technology, Agency of Industrial Science & Technology. This microorganism can be stored according to a conventional method for storing Actinomycetes. The strain SAM 2084 has the following mycological properties.

(a) Morphological properties

The substrate mycelia of strain SAM 2084 are lengthily elongated, well-branched, and are not fragmented under conventional conditions.

The aerial mycelia are richly adhered on starch agar, glycerol-asparagine agar, or yeast-malt agar, and have good spore formation. The branch of the aerial mycelia is of typical trochoid branch type. The tip of the branch is in the form of a sake bottle, and 10 to 20 linear spore linkages are adhered. The observation of the aerial mycelia under an electron microscope shows that the spores are in a cylindrical form and have a size of 0.5 to 0.6 x 1.5 to 2.0 µm, the surfaces are smooth, and about 10 to 20 spores are generally linked. Sclerotia, sporangia and zoospores are not observed.

(b) Growth state on various media

The growth state on various agar media is as shown in Table 1 below. In the table, the standard shown in parentheses in the column of color is one described in "Color Harmony Manual" issued by Container Corporation of America. The observation was carried out after cultivation at 28°C for 14 to 21 days.

Table 1

| Growth state on various media | | | |
|---|---|---|---|
| Medium | Growth/reverse side color | Property of aerial mycelia/color | Soluble pigment |
| Sucrose nitrate agar | poor/colorless | None | None |
| Glucose-asparagine agar | Good/colorless | None | None |
| Glycerol-asparagine agar | Good/dark brown | Abundant, cottony/ivory (1 1/2ec) | Light brown |
| Starch agar | Good/light brown | Abundant, cottony/ivory (1 1/2ec) | None |
| Oatmeal agar | Moderate/light brown | Poor/light gray (1dc) | None |
| Yeast-malt agar | Good/dark brown | Abundant, cottony/ grayish green (1 1/2ge) | Dark brown |
| Tyrosine agar | Poor/colorless | None | None |
| Nutrient agar | Moderate/ colorless | None | None |
| Calcium malate agar | Poor/colorless | None | None |
| Bennett's agar | Good/light brown | Abundant, cottony/ grayish green (1 1/2ge) | Light brown |

(c) Physiological properties

(1) Growth temperature: The microorganism is grown in a yeast-starch agar in the temperature range of 15 to 41°C, and satisfactorily grown around 30°C.
(2) Liquefaction of gelatin: Positive
(3) Hydrolysis of starch: Positive
(4) Reduction of nitrate: Positive
(5) Peptonization of skimmilk: Positive Coagulation of skimmilk: Negative
(6) salt resistance: The microorganism is grown in a 1.5% NaCl-containing medium, whereas the growth is strongly inhibited at a NaCl concentration of not less than 3%.
(7) Production of melanin-like pigment: Negative

(d) Utilization of carbon source (ISP-9 medium used)

(1) Carbon source utilized: D-Glucose, D-fructose, glycerol, xylose, D-mannitol, myo-inositol, sucrose, L-arabinose
(2) Carbon source not utilized: L-Rhamnose, raffinose

(e) Cell analysis

The analysis was carried out by the method of Becker et al. (Appl. Microbiol. 13:236, 1965). As a result, diaminopimelic acid in the hydrolyzate of the whole cell was in LL form.

(f) Mycological properties

From the above properties, the strain SAM 2084 can be summarized as a strain which belongs to the genus Streptoverticillium among Actinomycetes and wherein the color tone of the aerial mycelia is "Yellow to Green" series, the branch of the aerial mycelia is of trochoid type, the spore linkage is linear, the surface of the spore is smooth, the reverse side color of the colony is light brown to black brown, and brownish soluble pigments are formed.
The strain having the above mycological properties, as compared with the description of species belonging to the genus Streptoverticillium, is considered to be a relative of Streptoverticillium morookaense. The present strain, however, is different from Streptoverticillium morookaense in several physiological properties, and is designated as

Streptoverticillium sp. SAM 2084.

The compound represented by formula (I) can be produced by culturing the microorganism, capable of producing the contemplated compounds, belonging to the genus Streptoverticillium. For example, these compounds may be produced by culturing Streptoverticillium sp. SAM 2084 to produce and accumulate the compound represented by formula (I) and purifying the compound represented by formula (I) from the culture medium and/or culture cells by conventional purification means.

In conventional culturing, the compound represented by formula (I) is produced in the form of a mixture of analogues such that various hydrocarbon groups have been introduced into $R^1$. Therefore, in order to produce various compounds represented by formula (I), a fatty acid corresponding to a desired aliphatic hydrocarbon group $R^1$ or a soluble salt thereof, such as a sodium, potassium, or ammonium salt thereof, is preferably added to the culture medium. For example, the addition of the above material preferably in an amount of 1 to 100 ppm, more preferably 1 to 10 ppm, results in the production of a compound represented by formula (I) wherein $R^1$ represents a desired aliphatic hydrocarbon group.

In the production of the compound represented by formula (I), the medium used in the culturing of the Actinomycetes may be any of liquid and solid media. In general, however, shaking culture or aeration agitation culture in a liquid medium is advantageous.

The medium used is not particularly limited so far as the microorganism capable of producing the contemplated compound can be satisfactorily grown to accumulate the compound represented by formula (I). More specifically, carbon sources usable herein include saccharides, which the microorganism assimilate, for example, glucose, lactose, glycerin, starch, sucrose, dextrin, and syrup. Nitrogen sources usable herein include, for example, organic nitrogen sources, for example, protein hydrolyzates, such as polypeptone and casamino acid, meat extract, yeast extract, soybean meal, corn steep liquor, and amino acids, and inorganic nitrogen sources, such as ammonium salts and nitrates.

Further, the following additives may be added as optional components to the medium. For example, various inorganic salts, such as phosphates, magnesium nitrate, sodium chloride, and calcium carbonate, may be added for the regulation of osmotic pressure, the adjustment of pH, the supplement of minor components and other purposes. Various vitamins, nucleic acid-related compounds and the like may also be added from the viewpoint of accelerating the growth of the microorganism. During the culturing period, anti-foaming agents, such as silicones, polypropylene glycol derivatives, soybean oil, and the like, may be added.

In the culturing, according to a conventional method, main culturing is preferably carried out using a culture obtained by previous culturing on a small scale. In the main culturing, culturing conditions, such as culturing temperature, culturing period, pH of culture medium, and the quantity of airflow, should be properly selected and regulated so that the amount of the compound represented by formula (I) accumulated becomes the maximum value.

More specifically, regarding aeration conditions, the quantity of airflow is preferably 0.5 to 2 vvm, more preferably about 0.5 to 1 vvm, the culturing temperature is typically 15 to 41°C, preferably 20 to 37°C, more preferably 25 to 30°C, the culturing period is preferably 2 to 3 days, and the culture medium is preferably around neutral.

In the above culturing, the compound represented by formula (I) is accumulated in both the culture solution and the mycelia. Therefore, the compound represented by formula (I) can be extracted from the culture solution with a water-insoluble organic solvent, such as ethyl acetate, chloroform, or dichloromethane. On the other hand, in the extraction of the compound represented by formula (I) from the culture mycelia, the compound represented by formula (I) can be extracted with a solvent which can break cell walls of mycelia, such as acetone, directly from cells harvested, for example, by filtration or centrifugation. When the culture mycelia have been crumbled by means of glass beads or the like, the compound represented by formula (I) may be extracted in the same manner as used in the extraction from the culture medium.

Conventional purification methods may be used in the isolation and purification of the compound represented by formula (I) from the crude extract. For example, the isolation and purification may be carried out by combining various purification means, for example, solvent transfer dissolution, normal phase and reversed phase column chromatographies, gel filtration chromatography, and crystallization.

Since the compound represented by formula (I) according to the present invention is produced in the form of a mixture of analogues wherein various hydrocarbon groups have been introduced into $R^1$, the use of normal phase or reversed phase high performance liquid chromatography (HPLC) is particularly useful in the isolation and purification of the analogues.

UK-2A to D and UK-3A can be specifically isolated and purified according to the method described in Japanese Patent Laid-Open No. 233165/1995 or J. Antibiotics 50: 551-555 (1997).

Agents and methods controlling for rice blast and wheat leaf rust

The rice blast and the wheat leaf rust control agents according to the present invention comprise, as an active ingredient, the compound represented by formula (I). The compound represented by formula (I) is preferably

added as a compound, which has been isolated and purified to some extent, to the agent. Alternatively, however, the microorganism capable of producing the compound represented by formula (I) or the culture product thereof may be added as the active ingredient to the agent.

Preferably, the control agent according to the present invention is provided in such a manner that a carrier is used and, in addition, if necessary, proper adjuvants are compounded followed by provision of a suitable formulation. Preferably, for example, the agent can be formulated into oil mediums, emulsifiable concentrates, dust, wettable powder, granules, and suspension concentrates, which are suitably diluted before use.

Preferred carriers usable herein include: solid powder or particulate carriers, such as clay, talc, diatomaceous earth, white clay, calcium carbonate, silicic anhydride, bentonite, sodium sulfate, silica gel, salts of organic acids, saccharides, starch, resins, and synthetic or naturally occurring polymers; and liquid carriers, for example, aromatic hydrocarbons, such as xylene, aliphatic hydrocarbons, such as kerosene, ketones, such as methyl ethyl ketone, cyclohexanone, and isophorone, lactams, ethers, such as anisole, alcohols, such as ethanol, propanol, and ethylene glycol, esters, such as ethyl acetate and butyl acetate, dimethylsulfoxide, dimethylformamide, and water.

In order to more surely attain the effect of the control agent, preferably, the agent is used in combination with adjuvants properly selected, depending upon applications, from emulsifiers, dispersants, wetting agents, binders, and lubricants. Adjuvants usable herein include, for example, nonionic and ionic surfactants, carboxymethylcellulose, polyvinyl acetate, polyvinyl alcohol, gums, salts of stearate, waxes, and adhesive agents.

In the control agent according to the present invention, the compound represented by formula (I) is generally incorporated in an amount of about 0.01 to 10% by weight, preferably about 0.1 to 5% by weight, for dust formulation, in an amount of about 1 to 90% by weight, preferably about 5 to 75% by weight, for wettable powder, in an amount of about 0.01 to 40% by weight, preferably about 0.1 to 20% by weight, for granules, in an amount of about 1 to 60% by weight, preferably about 5 to 40% by weight, for liquid formulation, and in an amount of about 1 to 80% by weight, preferably about 5 to 50% by weight, for suspension concentrate.

The rice blast and wheat leaf rust control agents according to the present invention can be, of course, used alone, or alternatively can be used in combination with or as a mixture with, for example, fungicides, insecticides, herbicides, plant growth regulators, or fertilizers, for example, Blasticidin S, Kasugamycin, Tricyclazole, Pyroquilone, Isoprothiolane, and Fthalide, Probenazole; methoxyacrylates, benzothiadiazoles, cyclopropanes, pyrimidines, dithiocarbamates, carbamates, benzoimidazoles, benzothiazoles, triazines, cyanoacetic amides, imidazoles, pyrroles, nicotinoides, guanidines, nitromethylenes, cyanomethylenes, isothiazoles, anilides, quinolines, organochlorines, sulfonic amides, pyrethroids, sulfonylureas, ureas, and organophosphorus compounds.

The method for controlling rice blast or wheat leaf rust according to the present invention comprises the step of applying the compound represented by formula (I) to a rice or wheat plant per se, seeds thereof, a field in which the rice or wheat plant per se is being grown or to be grown, or an instrument utilized in the growth of the rice or wheat plant. More specifically, when the incidence of rice blast or wheat leaf rust is forecasted, the compounds represented by formula (I) may be applied beforehand to stems and leaves of the plant, or alternatively may be applied to roots of the plant, the surface of field, water surface, side dressing (i.e. what is called "sokujou" in Japanese), soil, seeds, nursery boxes or the like. Further, the use of the compound represented by formula (I) after the incidence and spreading of the disease as a result of infection with phathogenic fungus which cause rice blast or wheat leaf rust, can eliminate or inhibit the diseases.

The agent according to the present invention may be applied in an amount which is properly determined by taking into consideration the form of preparations and the method, purpose and season of the spraying. In general, however, the amount of the control agent applied in terms of the amount of the compound represented by formula (I) as an active ingredient is preferably 10 to 2,000 g per ha, more preferably 50 to 1,000 g, for control of rice blast, and is preferably 5 to 1,000 g per ha, more preferably 10 to 500 g, for control of wheat leaf rust.

## EXAMPLES

The following preparation examples and test examples further illustrate the present invention, but are not intended to limit it.

UK-2A, UK-2B, UK-2C, UK-2D, and UK-3A used in the examples were produced according to the production process of Japanese Patent Laid-Open No. 233165/1995 or J. Antibiotics 50: 551-555 (1997), and isolated and purified.

Preparation 1: Preparation of wettable powder

UK-2A (10 parts by weight), 5 parts by weight of white carbon, 20 parts by weight of a polyoxyethylene alkyallyl ether, and 65 parts by weight of clay were homogeneously ground and mixed together to prepare a wettable powder containing 10% of UK-2A. The wettable powder may be used, for example, by, after dilution of the wettable powder by 1,000 to 100,000 times with water or the like, spraying of or immersion in the diluted agent.

Preparation 2: Preparation of dust

UK-2A (0.3 part by weight), 0.5 part by weight of magnesium stearate, 59.2 parts by weight of talc, and 40 parts by weight of clay were homogeneously ground and mixed together to prepare a dust containing 0.3% of UK-2A. This dust as such may be applied, for example, by means of a power duster, a pipe duster, an aircraft applicator or the like, or alternatively may be used in soil treatment or in dust coating of seeds.

Preparation 3: Preparation of granules

UK-2A (10 parts by weight), 79 parts by weight of clay, 10 parts by weight of calcium ligninsulfonate, and 1 part by weight of polyvinyl alcohol were homogeneously ground and mixed together. Water was then added to the mixture, followed by kneading. The kneaded product was granulated, and the granules were dried to prepare granules containing 10% of UK-2A. The granules may be applied to a cultivation field for spraying thereof to the surface of water, or for application to nursery boxes, soil treatment or other purposes.

Preparation 4: Preparation of liquid formulation (1)

UK-2A (2 parts by weight), 10 parts by weight of δ-valerolactam, 10 parts by weight of a polyoxyethylene alkyl sulfate, 5 parts by weight of a polyoxyethylene alkylallyl ether, and 73 parts by weight of cyclohexanone were homogeneously mixed and dissolved together to prepare a liquid formulation containing 2% of UK-2A. This liquid formulation may be used, for example, by, after proper dilution of the liquid formulation with water, spraying of or immersion in the diluted liquid.

Preparation 5: Preparation of liquid formulation (2)

UK-2A (3 parts by weight), 77 parts by weight of isophorone, and 20 parts by weight of a polyoxyalkyl sulfate were homogeneously mixed and dissolved together to prepare a liquid formulation containing 3% of UK-2A. This liquid formulation may be used, for example, by, after proper dilution of the liquid formulation with water, spraying of or immersion in the diluted liquid.

Example 1: Control of rice blast

Eight fourth-leaf stage rice seedling (variety: Jikkoku) grown in each of plastic pots containing cultivation soil were provided as a test plant.

Liquid formulations were prepared wherein UK-2A, UK-2B, UK-2C, UK-2D, or UK-3A prepared as described in Preparation Example 4 was contained alone. Separately, a Rabcide sol, a commercially available fungicide for agricultural and horticultural applications, was provided. They were diluted with water to bring the final concentration to 5.0 ppm to prepare liquid preparations.

Each of the liquid preparations thus prepared was applied in an amount of 10 ml per three pots by means of a spray gun to stems and leaves of the test plant. The liquid preparation on the plant was air-dried. Thereafter, the pots were evenly inoculated by spraying with a conidial suspension ($1 \times 10^6$/ml) of rice blast fungi (Pyricularia oryzae), which had been previously cultured in an oatmeal-agar medium (5% oatmeal, 1% sucrose, and 1% agar) at 28°C for 8 days. The pots were then kept in a moist chamber at 24°C overnight. Then, they were transferred to an environment controlled greenhouse kept at 18°C at night and at 25°C in the daytime to induce the disease. Seven days after the inoculation, the number of lesions which had appeared in inoculated leaves were counted. The average number of lesions per rice seedling in the treated plot was determined, and the protective value was calculated by the following equation. As a control, a conidial suspension of rice blast fungi was inoculated by spraying in the same manner as described above, except that the liquid preparation was not applied. The average number of lesions per rice seedling in the nontreated plot, in which the disease had been induced, was then determined, and the protective value was calculated by the following equation in the same manner as described above.

$$\text{Protective value} = (1 - \text{average number of lesions in treated plot/average number of lesions in nontreated plot}) \times 100$$

The results were as summarized in Table 2. Further, any phytotoxicity was not observed.

Table 2

| Test results on rice blast | | |
|---|---|---|
| Sample | Concentration(ppm) | Protective value |
| Non-treated | - | 0 |
| UK-2A | 5.0 | 100 |
| UK-2B | 5.0 | 100 |
| UK-2C | 5.0 | 100 |
| UK-2D | 5.0 | 100 |
| UK-3A | 5.0 | 100 |
| Rabcide sol | 5.0 | 57 |

Example 2: Control of wheat leaf rust

Fourth-leaf stage wheat seedling (variety: Norin No. 61) grown in each of plastic pots containing cultivation soil were provided as a test plant.

Liquid formulations were prepared wherein UK-2A, UK-2B, UK-2C, UK-2D, or UK-3A prepared as described in Preparation Example 4 was contained alone. Separately, a wettable powder of Bayleton, a commercially available fungicide for agricultural and horticultural applications, was provided. They were diluted with water to bring the final concentration to 10 ppm to prepare liquid preparations

Each of the liquid preparations thus prepared was applied in an amount of 10 ml per three pots by means of a spray gun to stems and leaves of the test plant. The liquid preparation on the plant was air dried. Thereafter, the pots were evenly inoculated by spraying with an urediniospore suspension (1 x $10^6$/ml) of wheat leaf rust fungi (Puccinia recondita), which had been prepared after the harvest of wheat leaves in which leaf rust had been previously induced. The pots were then kept in a moist chamber at 21°C overnight. Then, they were transferred to an environment controlled greenhouse kept at 18°C at night and at 22°C in the daytime to induce the disease. Fourteen days after the inoculation, the area of the disease which had appeared in inoculated leaves was investigated. The results were expressed in terms of index according to the following criteria, and the disease rate of the treated plot was determined by the following equation, and the protective value was calculated. Separately, an urediniospore suspension of wheat leaf rust fungi was inoculated in the same manner as described above, except that the liquid preparation was not applied. Thus, the disease was induced. The protective value was calculated from the disease rate in the nontreated plot in the same manner as described above.

Index
- 0: No lesion occurred.
- 1: Several lesions occurred.
- 2: The disease area was less than one-fourth of the leaf area.
- 3: The disease area was one-fourth to less than a half of the leaf area.
- 4: The disease area was not less than a half to three-fourth of the leaf area.
- 5: The disease area was not less than three-fourth of the leaf area.

$$\text{The disease rate} = \Sigma\,(\text{index x number of plants for each severity})/(\text{number of investigated plants x 5}) \times 100$$

$$\text{Protective value} = (1 - \text{disease rate in treated plot/disease rate in nontreated plot}) \times 100$$

The results were as summarized in Table 3. Further, any phytotoxicity was not observed.

Table 3

| Test results on wheat leaf rust | | |
|---|---|---|
| Sample | Concentration(ppm) | Protective value |
| Non-treated | - | 0 |
| UK-2A | 10 | 100 |
| UK-2B | 10 | 100 |
| UK-2C | 10 | 100 |
| UK-2D | 10 | 100 |
| UK-3A | 10 | 100 |
| Wettable powder of Bayleton | 10 | 60 |

## Claims

1. A rice blast control agent, comprising a compound represented by formula (1):

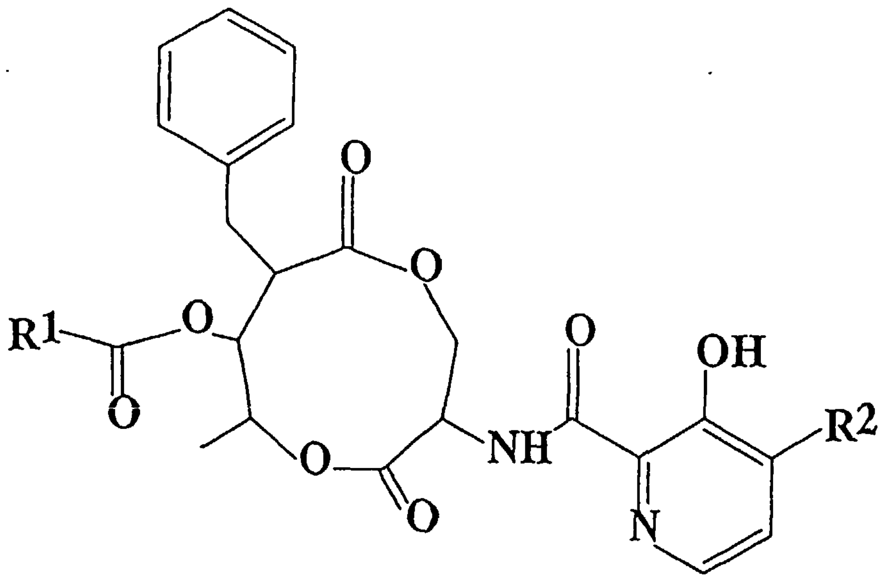

(I)

wherein

$R^1$ represents straight-chain or branched saturated aliphatic hydrocarbon group or straight-chain or branched unsaturated aliphatic hydrocarbon group; and
$R^2$ represents a hydrogen atom or methoxy group.

2. The rice blast control agent according to claim 1, wherein $R^1$ represents isopropyl group, (Z)-2-butenyl group, isobutyl group, or sec-butyl group and $R^2$ represents a hydrogen atom or methoxy group.

3. A method for controlling rice blast, comprising the step of applying the compound represented by formula (I) defined in claim 1 to a rice plant per se, seeds thereof, a field in which the rice plant is being grown or to be grown, or an instrument utilized in the growth of the rice plant.

**4.** The method according to claim 3, wherein the compound represented by formula (I) is applied to stems and leaves of the rice plant, or alternatively is used for treatment of roots of the rice plant, field treatment, water surface treatment, side dressing treatment, soil treatment, rice seed treatment, or treatment of a rice nursery box.

**5.** A wheat leaf rust control agent, comprising the compound represented by formula (I) according to claim 1.

**6.** The wheat leaf rust control agent according to claim 5, wherein $R^1$ represents isopropyl group, (Z)-2-butenyl group, isobutyl group, or sec-butyl group and $R^2$ represents a hydrogen atom or methoxy group.

**7.** A method for controlling wheat leaf rust, comprising the step of applying the compound represented by formula (I) according to claim 1 to a wheat plant per se, seeds thereof, a field in which the wheat plant is being grown or to be grown, or an instrument utilized in the growth of the wheat plant.

**8.** The method according to claim 7, wherein the compound represented by formula (I) is applied to stems and leaves of the wheat plant, or alternatively is used for treatment of roots of the wheat plant, wheat field treatment, side dressing treatment, soil treatment, wheat seed treatment, or treatment of a wheat nursery box.

## INTERNATIONAL SEARCH REPORT

International application No.
PCT/JP98/03876

**A. CLASSIFICATION OF SUBJECT MATTER**
Int.Cl$^6$ A01N43/40

According to International Patent Classification (IPC) or to both national classification and IPC

**B. FIELDS SEARCHED**

Minimum documentation searched (classification system followed by classification symbols)
Int.Cl$^6$ A01N43/40

Documentation searched other than minimum documentation to the extent that such documents are included in the fields searched

Electronic data base consulted during the international search (name of data base and, where practicable, search terms used)
CA (STN), REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | JP, 7-233165, A (Suntory Ltd.), 5 September, 1995 (05. 09. 95), Claims 1, 2 ; page 5, left column, lines 36 to 44 (Family: none) | 1-8 |
| X | UEKI et al., "UK-3A, a novel antifungal antibiotic from Streptomyces sp.517-02: fermentation, isolation, structural elucidation and biological properties". Journal of Antibiotics, 1997, Vol. 50(7), p.551-555. | 1-8 |
| X | HANAFI et al., "UK-2A, B, C and D, novel antifungal antibiotics from Streptomyces sp.517-02. II. Structural elucidation". Journal of Antibiotics, 1996, Vol. 49(12), p.1226-1231. | 1-8 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 12 November, 1998 (12. 11. 98) | 24 November, 1998 (24. 11. 98) |
| Name and mailing address of the ISA/ Japanese Patent Office | Authorized officer |
| Facsimile No. | Telephone No. |

Form PCT/ISA/210 (second sheet) (July 1992)